(19)

Europäisches
Patentamt
European
Patent Office
Office européen
des brevets

(11) **EP 2 871 189 A1**

(12) **EUROPEAN PATENT APPLICATION**

(43) Date of publication:
**13.05.2015 Bulletin 2015/20**

(51) Int Cl.:
***C07K 16/12*** *(2006.01)*       ***G01N 33/53*** *(2006.01)*

(21) Application number: **13306527.6**

(22) Date of filing: **07.11.2013**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**

(71) Applicants:
• **INSTITUT PASTEUR
75015 Paris (FR)**
• **Centre National de la Recherche Scientifique
(CNRS)
75016 Paris (FR)**

(72) Inventors:
• **Girard-Blanc, Christine
92130 Issy Les Moulineaux (FR)**

• **Krey, Thomas
78190 Trappes (FR)**
• **Vasiliauskaite, Ieva
75016 Paris (FR)**
• **Nato, Farida
92160 Antony (FR)**
• **Lafaye, Pierre
92240 Malakoff (FR)**
• **Dartevelle, Sylvie
78640 Neauphle-Le-Chateau (FR)**
• **Rey, Felix
91190 Gif-sur-Yvette (FR)**

(74) Representative: **Regimbeau
20, rue de Chazelles
75847 Paris Cedex 17 (FR)**

(54) **High-affinity monoclonal anti-strep-tag antibody**

(57)    The present invention provides novel antibodies recognizing the so-called "Strep-Tag II" peptide, to its preparation and to uses thereof. Anti-strep tag antibodies are useful for quick purification but also for sensitive detection and tight immobilization of Strep-Tag II fusion proteins.

EP 2 871 189 A1

SEQUENCE LISTING

<110> INSTITUT PASTEUR

<120> HIGH-AFFINITY MONOCLONAL ANTI-STREP-TAG ANTIBODY

<130> 70121 D31349

<160> 13

<170> PatentIn version 3.5

<210> 1
<211> 10
<212> PRT
<213> artificial

<220>
<223> CDR1 of Light Chain

<400> 1

Glu Ser Val Asp Ser Tyr Gly Lys Ser Phe
1               5                   10


<210> 2
<211> 3
<212> PRT
<213> artificial

<220>
<223> CDR2 of Light Chain

<400> 2

Arg Ala Ser
1


<210> 3
<211> 9
<212> PRT
<213> artificial

<220>
<223> CDR3 of Light Chain

<400> 3

Gln Gln Asn Asn Glu Asp Pro Trp Thr
1               5


<210> 4
<211> 8
<212> PRT
<213> artificial

<220>
<223> CDR1 of Heavy Chain

<400> 4

Gly Tyr Thr Phe Thr Asn Tyr Tyr
1               5

<210>   5
<211>   8
<212>   PRT
<213>   artificial

<220>
<223>   CDR2 of Heavy Chain

<400>   5

Leu Asn Pro Asn Asn Gly Asp Thr
1               5

<210>   6
<211>   13
<212>   PRT
<213>   artificial

<220>
<223>   CDR3 of Heavy Chain

<400>   6

Ala Arg Thr Gly Arg Tyr Glu Glu Asn Ala Met Asp Tyr
1               5                   10

<210>   7
<211>   111
<212>   PRT
<213>   artificial

<220>
<223>   light chain

<400>   7

Glu Leu Val Met Thr Gln Ser Pro Ala Ser Leu Ala Val Ser Leu Gly
1               5                   10                  15

Gln Arg Ala Thr Ile Ser Cys Arg Ala Ser Glu Ser Val Asp Ser Tyr
            20                  25                  30

Gly Lys Ser Phe Met His Trp Tyr Gln Leu Lys Pro Gly Gln Pro Pro
            35                  40                  45

Lys Leu Leu Ile Tyr Arg Ala Ser Asn Leu Glu Ser Gly Val Pro Ala
        50                  55                  60

Arg Phe Ser Gly Ser Gly Ser Arg Thr Asp Phe Thr Leu Thr Ile Asp
65                  70                  75                  80

Pro Val Glu Ala Asp Asp Ala Ala Thr Tyr Tyr Cys Gln Gln Asn Asn
                85                      90                      95

Glu Asp Pro Trp Thr Phe Gly Gly Gly Thr Lys Leu Glu Ile Lys
                100                     105                     110

```
<210>   8
<211>   120
<212>   PRT
<213>   artificial

<220>
<223>   heavy chain

<400>   8
```

Glu Val Gln Leu Glu Gln Ser Gly Pro Glu Leu Val Lys Pro Gly Ala
1                   5                   10                  15

Ser Val Lys Met Ser Cys Lys Ala Ser Gly Tyr Thr Phe Thr Asn Tyr
                20                      25                      30

Tyr Met Lys Trp Val Lys Gln Ser His Gly Lys Ser Leu Glu Trp Ile
                35                      40                      45

Gly Asp Leu Asn Pro Asn Asn Gly Asp Thr Phe Tyr Asn Gln Lys Phe
        50                      55                      60

Lys Gly Lys Ala Thr Leu Thr Val Asp Lys Ser Ser Asn Thr Ala Tyr
65                      70                      75                      80

Met Gln Leu Asn Ser Leu Thr Ser Glu Asp Ser Ala Val Tyr Tyr Cys
                85                      90                      95

Ala Arg Thr Gly Arg Tyr Glu Glu Asn Ala Met Asp Tyr Trp Gly Gln
                100                     105                     110

Gly Thr Ser Val Thr Val Ser Ser
        115                     120

```
<210>   9
<211>   8
<212>   PRT
<213>   artificial

<220>
<223>   minimal sequence of Strep Tag II peptide

<400>   9
```

Trp Ser His Pro Gln Phe Glu Lys
1                   5

```
<210>    10
<211>    10
<212>    PRT
<213>    artificial

<220>
<223>    recognized sequence of Strep Tag II peptide

<400>    10

Ser Ala Trp Ser His Pro Gln Phe Glu Lys
1                5                   10


<210>    11
<211>    30
<212>    PRT
<213>    artificial

<220>
<223>    double strep-tag II peptide

<400>    11

Ala Gly Trp Ser His Pro Gln Phe Glu Lys Gly Gly Gly Ser Gly Gly
1                5                   10                  15


Gly Ser Gly Gly Gly Ser Trp Ser His Pro Gln Phe Glu Lys
            20          25                  30


<210>    12
<211>    252
<212>    PRT
<213>    artificial

<220>
<223>    recombinant ectodomain of glycoprotein E2 of GB virus

<400>    12

Asn Pro Ile Arg Val Pro Thr Gly Cys Ser Ile Ala Glu Phe Cys Ser
1                5                   10                  15


Pro Leu Met Ile Pro Cys Pro Cys His Ser Tyr Leu Ser Glu Asn Val
            20                  25                  30


Ser Glu Val Ile Cys Tyr Ser Pro Lys Trp Thr Arg Pro Ile Thr Leu
            35                  40                  45


Glu Tyr Asn Asn Ser Ile Ser Trp Tyr Pro Tyr Thr Ile Pro Gly Ala
        50                  55                  60


Arg Gly Cys Met Val Lys Phe Lys Asn Asn Thr Trp Gly Cys Cys Arg
65                  70                  75                  80
```

16

```
Ile Arg Asn Val Pro Ser Tyr Cys Thr Met Gly Thr Asp Ala Val Trp
                85              90                  95

Asn Asp Thr Arg Asn Thr Tyr Glu Val Cys Gly Val Thr Pro Trp Leu
            100             105                 110

Thr Thr Ala Trp His Asn Gly Ser Ala Leu Lys Leu Ala Ile Leu Gln
        115                 120                 125

Tyr Pro Gly Ser Lys Glu Met Phe Lys Pro His Asn Trp Met Ser Gly
    130                 135                 140

His Leu Tyr Phe Glu Gly Ser Asp Thr Pro Ile Val Tyr Phe Tyr Asp
145                 150                 155                 160

Pro Val Asn Ser Thr Leu Leu Pro Pro Glu Arg Trp Ala Arg Leu Pro
                165                 170                 175

Gly Thr Pro Pro Val Val Arg Gly Ser Trp Leu Gln Val Pro Gln Gly
            180                 185                 190

Phe Tyr Ser Asp Val Lys Asp Leu Ala Thr Gly Leu Ile Thr Lys Asp
        195                 200                 205

Lys Ala Trp Lys Asn Tyr Gln Phe Glu Asp Asp Asp Asp Lys Ala Gly
    210                 215                 220

Trp Ser His Pro Gln Phe Glu Lys Gly Gly Gly Ser Gly Gly Gly Ser
225                 230                 235                 240

Gly Gly Gly Ser Trp Ser His Pro Gln Phe Glu Lys
            245                 250
```

```
<210>  13
<211>  12
<212>  PRT
<213>  Artificial Sequence

<220>
<223>  simple strep tag peptide

<400>  13
```

```
Leu Glu Ser Ala Trp Ser His Pro Gln Phe Glu Lys
1               5                   10
```

**Description**

**FIELD OF THE INVENTION**

The present invention provides novel antibodies recognizing the so-called "Strep-Tag II" peptide, to its preparation and to uses thereof. Anti-strep tag antibodies are useful for quick purification but also for sensitive detection and tight immobilization of Strep-Tag II fusion proteins.

**BACKGROUND OF THE INVENTION**

In response to the rapidly growing field of proteomics, the use of recombinant polypeptides has increased greatly in recent years. Recombinant hybrids containing a polypeptide fusion partner, hereafter termed "peptide tag", so as to facilitate the purification of target polypeptides are widely used. Many different proteins, domains, or peptides can be fused with a target polypeptide. The advantages of using fusion polypeptide to facilitate purification, screening, sorting and detection of recombinant polypeptides are well recognized. The most frequently used and interesting systems are reviewed in Terpe K., Appl. Microbiol. Biotechnol. (2003) and more recently in Arnau J. et al, Protein Expr. Purif. (2006). They are for example Arg-tag, calmodulin-binding peptide, cellulose-binding domain, DsbA, c-myc-tag, glutathione S-transferase, FLAG-tag, HAT-tag, His-tag, maltose-binding protein, NusA, S-tag, SBP-tag, Strep-tag, and thioredoxin.

Use of antibodies specific for these peptide tags is a widely used strategy for purification purposes and/or for coating those polypeptides on various solid supports. A number of antibodies recognizing these peptide tags are already commercially available (GST-specific antibody from Invitrogen; anti-FLAG(TM) antibodies from Sigma-aldrich Co, Anti-Strep-Tag(TM) antibodies from IBA and the like).

The Strep-Tag technology

Streptavidin is a tetrameric protein purified from *Streptomyces avidinii*. It is widely used in numerous molecular biological protocols due to its strong affinity for biotin. The so-called "Strep-Tag" is a nine amino acid-peptide that binds specifically to streptavidin and occupies the same pocket where biotin is normally complexed. Since the Strep-tag participates in a reversible interaction, it can be applied for the efficient purification of corresponding fusion proteins on affinity columns with immobilized streptavidin. Elution of the bound recombinant protein can be effected under mild buffer conditions by competition with biotin or a suitable derivative. In addition, Strep-Tagged fusion proteins can be easily detected in immunochemical assays, like Western blots or ELISAs, by means of commercially available streptavidin-enzyme conjugates. The Strep-Tag/streptavidin system has been systematically optimized over the past years, including the engineering of streptavidin itself. Structural insight into the molecular mimicry between the peptide and biotin was furthermore gained from X-ray crystallographic analysis. As a result the system provides a reliable and versatile tool in recombinant protein chemistry. Exemplary applications of the Strep-Tag are discussed in Skerra A and Schmidt TG (Biomol. Eng. 1999).

The Strep-Tag/streptavidin system has been optimized over the years, including X-ray crystallographic analysis of the streptavidin-Strep-Tag complex, development of an optimized Strep-Tag, called "Strep-Tag II", which permits greater flexibility in the choice of the attachment site (N- or C- terminal end of the polypeptide of interest), and engineering of a variant streptavidin with improved Strep-Tag-binding capacity, called "Strep-Tactin". As a result, the Strep-Tag today provides a reliable tool in recombinant protein chemistry, which has proven particularly useful for the parallel isolation and functional analysis of multiple gene products in proteome research (Skerra A & Schmidt TG, Methods Enzymol. 2000).

The II peptide

Similarly as with other peptide tags such as the His$_6$ tag, the calmodulin-binding peptide or the Flag tag, the Strep-Tag II peptide (hereafter referred to as "Strep-Tag II") can be easily fused to a recombinant polypeptide during subcloning of its cDNA or gene. A particular benefit of the Strep-Tag II is that it does not hamper protein folding or secretion and it usually does not interfere with protein function. Also, the Strep-Tag II is largely resistant to cellular proteases, it can be used in the presence of mild detergents and it is biochemically almost inert. This gives a clear advantage over the His$_6$-tag, the calmodulin-binding peptide and the Flag -tag, whose function moreover is metal ion dependent. Consequently, for most applications, there is no need to cleave the Strep-Tag II off the protein of interest. Notably, intact Strep-Tag II fusion proteins were successfully employed for protein crystallography in several cases (Schmidt T. & Skerra A., Nature protocols, 2007).

Strep-Tag II allows the isolation of sensitive proteins in a native state, but it is also possible to purify intact protein complexes in a preparative manner, even if only one subunit carries the tag (Skerra A & Schmidt TG, Methods Enzymol. 2000). Furthermore, the Strep-Tag II was successfully applied for the identification of protein interaction partners in proteomics research (Junttila M.R. et al, Proteomics 2005). In this context, the use of a tandem arrangement of two Strep-Tag II sequences appeared to be advantageous for higher purification yields of protein complexes, also allowing the application of elevated detergent concentrations to reduce background.

Based on its proprietary *Strep*-tag® technology, IBA recently developed the One-STrEP-tag (tandem *Strep*-tag®) for a mild and rapid purification of intact protein complexes on immobilized *Strep*-Tactin®. The One-STrEP-tag combines high specificity and mild conditions with higher affinity thereby enabling efficient purification in batch or directly from culture supernatants. Furthermore, it tolerates elevated detergent concentrations to reduce background.

<u>Antibodies recognizing the II peptide</u>

Of note, the affinity of the Strep-tag II peptide for an individual binding site (monovalent interaction) on Strep-Tactin is moderate, with a dissociation constant around 1 $\mu$M (Schmidt TG et al, J.Mol. Biol. 1996). Consequently, the formation of a stable complex between the naturally tetrameric streptavidin (or Strep-Tactin) and a Strep-Tag II fusion protein requires an avidity effect, which involves multivalency as well on the side of the recombinant protein. Although this moderate affinity for monovalent interaction is sufficient for chromatographic purposes, however, for many other applications, it is desirable to detect a Strep-Tag II fusion protein with high sensitivity, for example, on a western blot or via histochemical staining, or in order to stably immobilize it on a solid support. For these applications, several antibodies can be used - for example, the commercially available *Strep*MAB-Classic, monoclonal antibodies *Strep*MAB-Immo (IBA) or Strep-tag antibody (Qiagen). In particular, the high-affinity monoclonal antibody called "*Strep*MAB-Immo" allows the stable capturing of monovalent Strep-Tag II fusion proteins after immobilization to a solid matrix (e.g., a microtiter plate, paramagnetic beads or a surface plasmon resonance chip). These reagents work nicely for quick purification but also sensitive detection and tight immobilization of Strep-Tag II fusion proteins (Schmidt T. & Skerra A., Nature protocols, 2007).

However, all the existing monoclonal anti-Strep Tag II antibodies produced so far recognize specifically the peptide sequence **SA**WSHPQFEK (SEQ ID NO:10) but poorly the minimal sequence of the Strep-Tag II peptide WSHPQFEK (SEQ ID NO:9). In other terms, high affinity of the existing antibodies is achieved only when the two amino acid residues "SA" (Serine-Alanine) are present in the linker located between the recombinant protein and the Strep-Tag (see for example for the IBA antibodies, the Box 2 in Schmidt T. & Skerra A., Nature protocols, 2007). These two amino acids are absolutely required for technologies necessitating high affinity Strep-Tag recognition, for example Western Blotting or Immunohistochemistry methods, or in order to immobilize Strep-tagged fusion proteins on Biacore sensor chips for performing Surface Plasmon Resonance (SPR)-mediated protein interaction studies (if these two amino acid residues are missing or mutated, the affinity of the commercial anti-Strep Tag antibodies is weaker resulting in a faint immobilization of the Strep-Tagged proteins on the sensor chip so that the Biacore experiments are unsuccessful). However, a lot of expression vectors that are classically used for generating recombinant proteins encode the minimal sequence WSHPQFEK but not the peptide sequence **SA**WSHPQFEK. Consequently, the recombinant proteins generated by means of these vectors contain the minimal sequence WSHPQFEK, which is not bound by the existing commercial anti-Strep Tag II antibodies with sufficient affinity and can therefore not be used in technologies necessitating high affinity Strep-Tag recognition, such as Biacore.

There is therefore a need of anti-Strep Tag II antibodies that present high affinity not only towards the peptide sequence **SA**WSHPQFEK, but also towards the minimal sequence WSHPQFEK (SEQ ID NO:9), independently of the upstream linker potentially located between the recombinant protein and the Strep-Tag II peptide.

In this context, the present inventors generated a monoclonal antibody, termed C23.21, which binds the Strep Tag peptide of sequence WSHPQFEK with picomolar affinity (as determined by ELISA experiments). Importantly, this antibody is the first antibody having a picomolar affinity towards the minimal sequence WSHPQFEK (SEQ ID NO:9), said affinity being independent of the amino acid sequence of the linker located between the recombinant protein and the Strep-Tag II peptide. Interestingly, it also has a high affinity towards the peptide sequence **SA**WSHPQFEK, and can therefore be used to recognize fusion proteins containing the peptide sequence WSHPQFEK whatever the sequence of the upstream linker is. As a matter of fact, the present inventors have shown that the monoclonal antibody C23.21 recognizes as efficiently fusion proteins containing either a single or a double Strep-Tag II peptide (having a sequence of **SA**WSHPQFEK or AGWSHPQFEKGGGSGGGSGGGSWSHPQFEK (SEQ ID NO:11), respectively) and detect these Strep-Tag II fusion proteins in ELISA and Western Blot analysis (see examples below).

On the contrary, the available anti-Strep Tag antibodies known so far have a low affinity with the minimal sequence WSHPQFEK (typically in the micromolar range) because they require the presence of a particular linker sequence upstream of the affinity tag (the two amino acids SA).

**FIGURES**

**Figure 1** discloses a western blot using the monoclonal antibody of the invention (1μg/mL) to detect the GBV-B glycoprotein E2 (lanes 4-6) and a control Fab (lanes 1-3) - both containing a double-Strep-Tag (DST) on a SDS-PAGE gel. This figure shows that both proteins are recognized by the monoclonal antibody of the invention with similar efficiency.

**Figure 2** discloses a SDS-PAGE analysis of crude extract, flow through, wash and elution fractions of the purification of an anti-GFAP camelid antibody fragment (VHH) E9 described in Perruchini et al, Acta Neuropathologica 2009 (left) and purified camelid antibody fragments (VHH) A12, G11, C7/G7 (right).

**Figure 3** discloses a Western Blot result of a purified Strep-Tagged VHH revealed with the monoclonal antibody of the invention.

**Figure 4** discloses the binding of Strep-tagged anti-GFAP VHH E9 (described in Perruchini et al, Acta Neuropathologica 2009) to its antigen GFAP in ELISA. The monoclonal antibody of the invention was used as a secondary antibody to detect binding.

**DETAILED DESCRIPTION OF THE INVENTION**

As mentioned above, the present inventors generated a monoclonal antibody, termed C23.21, which binds the Strep Tag peptide of sequence WSHPQFEK (SEQ ID NO:9) with picomolar affinity (as determined by ELISA experiments).

This antibody is actually a by-product of mouse immunization experiments conducted with the recombinant ectodomain of glycoprotein E2 of GB virus (GBV-B), containing a double Strep-Tag - i.e., two Strep-Tags separated by a linker peptide. Importantly, the binding of this antibody is entirely independent of the sequence of the linker located between the Strep Tag II peptide of sequence WSHPQFEK (SEQ ID NO:9) and the recombinant protein.

**The antibody of the invention**

In a first aspect, the present invention is therefore related to an antibody or a fragment thereof, which binds the peptide sequence represented by SEQ ID NO: 9 (Strep-Tag II), said antibody comprising six Complementary Determining Regions (CDRs) consisting of SEQ ID NO:1-6.

The term **"antibody"** as used herein is intended to include monoclonal antibodies, polyclonal antibodies, and chimeric antibodies. More particularly, an antibody (or "immunoglobulin") consists of a glycoprotein comprising at least two heavy (H) chains and two light (L) chains inter-connected by disulfide bonds.

Each heavy chain comprises a heavy chain variable region (or domain) (abbreviated herein as $V_H$) and a heavy chain constant region (hereafter $C_H$). Heavy chains are classified as gamma, mu, alpha, delta, or epsilon, and define the antibody's isotype as IgG, IgM, IgA, IgD, and IgE, respectively. The heavy chain constant region of the immunoglobulin IgG, IgD, and IgA (γ, δ and α chains respectively) comprises three domains (CH1, CH2, and CH3) and a hinge region for added flexibility, and the heavy chain constant region of the immunoglobulin IgM and IgE contains 4 domains (CH1, CH2, CH3, and CH4).

The antibody of the invention can be of the IgG, IgM, IgA, IgD, and IgE isotype, depending on the structure of its heavy chain. However, in a preferred embodiment, the antibody of the invention is of the IgG isotype, i.e., its heavy chain is of the gamma (γ) type.

IgG antibodies are classified in four distinct subtypes, named IgG1, IgG2, IgG3 and IgG4 in order of their abundance in serum (IgG1 being the most abundant). The structure of the hinge regions in the γ chain gives each of these subtypes its unique biological profile (even though there is about 95% similarity between their Fc regions, the structure of the hinge regions is relatively different).

The antibody of the invention can be of the IgG1, IgG2, IgG3 or IgG4 subtype. However, in a preferred embodiment, the antibody of the invention is of the IgG1 subtype.

Each light chain comprises a light chain variable region (abbreviated herein as $V_L$) and a light chain constant region comprising only one domain, $C_L$. There are two types of light chain in mammals: the kappa (κ) chain, encoded by the immunoglobulin kappa locus on chromosome 2, and the lambda (λ) chain, encoded by the immunoglobulin lambda locus on chromosome 22. In a preferred embodiment, the antibody of the invention has a Kappa light chain.

The $V_H$ and $V_L$ regions can be further subdivided into regions of hypervariability, termed "Complementarity Determining Regions" (CDR), which are primarily responsible for binding an epitope of an antigen, and which are interspersed with regions that are more conserved, termed "Framework Regions" (FR). Each $V_H$ and $V_L$ is composed of three CDRs and four FRs, arranged from amino-terminus to carboxy-terminus in the following order: FR1, CDR1, FR2, CDR2, FR3, CDR3, FR4. The assignment of amino acid sequences to each domain is in accordance with well-known conventions (CHOTHIA et al., J. Mol. Biol., 1987; CHOTHIA et al., Nature, 1989). The functional ability of the antibody to bind a particular antigen depends on the variable regions of each light/heavy chain pair, and is largely determined by the CDRs. The variable region of the heavy chain differs in antibodies produced by different B cells, but is the same for all antibodies produced by a single B cell or B cell clone (or hybridome).

By contrast, the constant regions of the antibodies mediate the binding of the immunoglobulin to host tissues or factors, including various cells of the immune system (e.g. effector cells) and the first component (Clq) of the classical complement system.

As used herein, the term **"antibody fragments"** intends to designate Fab, Fab', F(ab')2, scFv, dsFv, ds-scFv, dimers, minibodies, diabodies, and multimers thereof and bispecific antibody fragments. Antibodies can be fragmented using conventional techniques. For example, F(ab')2 fragments can be generated by treating the antibody with pepsin. The resulting F(ab')2 fragment can be treated to reduce disulfide bridges to produce Fab' fragments. Papain digestion can lead to the formation of Fab fragments. Fab, Fab' and F(ab')2, scFv, dsFv, ds-scFv, dimers, minibodies, diabodies, bispecific antibody fragments and other fragments can also be synthesized by recombinant techniques.

In a preferred embodiment, the antibody of the invention contains:

a) a light chain comprising three CDRs having the following amino acid sequences:

i) the light chain CDR1 (LC-CDR1): ESVDSYGKSF (SEQ ID NO:1);

ii) the light chain CDR2 (LC-CDR2): RAS (SEQ ID NO:2);

iii) the light chain CDR3 (LC-CDR3): QQNNEDPWT (SEQ ID NO: 3);
and

b) a heavy chain comprising three CDRs having the following amino acid sequences:

i) the heavy chain CDR1 (HC-CDR1): GYTFTNYY (SEQ ID NO: 4);

ii) the heavy chain CDR2 (HC-CDR2): LNPNNGDT (SEQ ID NO: 5);

iii) the heavy chain CDR3 (HC-CDR3): ARTGRYEENAMDY (SEQ ID NO: 6).

In a more preferred embodiment, the antibody of the invention comprises a light chain variable region ($V_L$) having the amino acid sequence SEQ ID NO: 7 and / or a heavy chain variable region ($V_H$) having the amino acid sequence SEQ ID NO: 8. In an even more preferred embodiment, the antibody of the invention comprises both a $V_L$ having the amino acid sequence SEQ ID NO: 7 and a $V_H$ having the amino acid sequence SEQ ID NO: 8.

In an even more preferred embodiment, the antibody of the invention is an IgG1 antibody having a Kappa light chain, comprising six Complementary Determining Regions (CDRs) consisting of SEQ ID NO:1-6, for example SEQ ID NO:1 to 3 for CDR1 to CDR3 (respectively) in the said Kappa light chain, and SEQ ID NO:4 to 6 for CDR1 to CDR3 (respectively) in its gamma heavy chain.

In an even more preferred embodiment, the antibody of the invention is an IgG1 antibody having a Kappa light chain having a $V_L$ of sequence SEQ ID NO: 7 and a gamma (y) heavy chain having a $V_H$ of sequence SEQ ID NO: 8.

The antibody of the invention is preferably a polyclonal or a monoclonal antibody.

A **"polyclonal antibody"** as used herein, designates antibodies that are obtained from different B cell resources. It typically includes various antibodies directed against various determinants, or epitopes, of the target antigen. These antibodies may be produced in animals. Conventional techniques of molecular biology, microbiology and recombinant DNA techniques are within the skill of the art. Such techniques are explained fully in the literature. For example, the antibodies of the invention may be prepared by the following conventional method. A mammal (e.g. a mouse, hamster, or rabbit) can be immunized with an immunogenic form of a protein carrying the SEQ ID NO:9, which elicits an antibody response in the mammal. Techniques for conferring immunogenicity on a polypeptide include conjugation to carriers or other techniques well known in the art. For example, the polypeptide can be administered in the presence of adjuvant. The progress of immunization can be monitored by detection of antibody titers in plasma or serum. Standard ELISA or other immunoassay procedures can be used with the immunogen as antigen to assess the levels of antibodies. Following immunization, antisera can be obtained and, if desired, polyclonal antibodies isolated from the sera.

A **"monoclonal antibody"**, as used herein, means an antibody arising from a nearly homogeneous antibody population. More particularly, the individual antibodies of a population are identical except for a few possible naturally-occurring mutations which can be found in minimal proportions. In other words, a monoclonal antibody consists of a homogeneous antibody arising from the growth of a single cell clone (for example a hybridoma, a eukaryotic host cell transfected with a DNA molecule coding for the homogeneous antibody, a prokaryotic host cell transfected with a DNA molecule coding for the homogeneous antibody, etc.) and is generally characterized by heavy chains of one and only one isotype and subtype, and light chains of only one type. Monoclonal antibodies are highly specific and are directed against a single antigen. In addition, in contrast with preparations of polyclonal antibodies, each monoclonal antibody is directed against a single epitope of the antigen.

In a preferred embodiment, the antibody of the invention is a monoclonal antibody.

To produce monoclonal antibodies, antibody producing cells (lymphocytes) can be harvested from the immunized animal as described above and fused with myeloma cells by standard somatic cell fusion procedures thus immortalizing these cells and yielding hybridoma cells. Such techniques are well known in the art (e.g. the hybridoma technique originally developed by Kohler and Milstein (Nature 256; 495- 497 (1975)) as well as other techniques such as the human B-cell hybridoma technique (Kozbor et al, Immunol Today 4 72 (1983)), the EBV-hybridoma technique to produce human monoclonal antibodies (Cole et al., Methods Enzymol, 121; 140-67 (1986)), and screening of combinatorial antibody libraries (Huse et al., Science 246; 1275 (1989)). Hybridoma cells can be screened immunochemically for production of antibodies specifically reactive with the target polypeptide so that only monoclonal antibodies binding to this polypeptide are isolated.

In some embodiments, recombinant antibodies are provided that bind the Strep-Tag II peptide of sequence SEQ ID NO:9. Recombinant antibodies include, but are not limited to, chimeric and humanized monoclonal antibodies, comprising both human and non-human portions, single-chain antibodies and multi- specific antibodies. A chimeric antibody is a molecule in which different portions are derived from different animal species, such as those having a variable region derived from a murine monoclonal antibody (mAb) and a human immunoglobulin constant region (See, e. g. , Cabilly et al. (US Pat No 4,816,567), and Boss et al. (US Pat No 4,816,397)). Single-chain antibodies have an antigen binding site and consist of single polypeptides. They can be produced by techniques known in the art, for example using methods described in Ladner et al. (US Pat No 4,946,778) or in Bird et al. (1988) Science 242 423-426). Multi-specific antibodies are antibody molecules having at least two antigen-binding sites that specifically bind different antigens. Such molecules can be produced by techniques known in the art, for example using methods described in Segal, US 4,676,980 or US 6,121,424. Monoclonal antibodies directed against the Strep-Tag II sequence SEQ ID NO:9 can be identified and isolated by screening a recombinant combinatorial immunoglobulin library (e.g., an antibody phage display library) with the polypeptide(s) of interest. Kits for generating and screening phage display libraries are commercially available. Additionally, examples of methods and reagents particularly amenable for use in generating and screening antibody display library can be found in, for example, US 5,223,409, WO 92/18619, and WO 91/17271.

Antibodies may be isolated after production (e.g., from the blood or serum of the animals) or synthetized and further purified by well-known techniques. Antibodies specific for a protein can be selected or purified by affinity chromatography, ELISPOT or ELISA. For example, a recombinantly expressed and purified (or partially purified) Strep-Tag II polypeptide may be produced, and covalently or non-covalently coupled to a solid support such as, for example, a chromatography column. The column can then be used to purify antibodies specific for the Strep-Tag II sequence SEQ ID NO:9, from a sample containing antibodies directed against a large number of different epitopes, thereby generating a substantially purified antibody composition, i.e., one that is substantially free of contaminating antibodies. By a "substantially purified antibody composition" it is meant, in this context, that the antibody sample contains at most only 30% (by dry weight) of contaminating antibodies directed against epitopes other than those of the Strep-Tag II sequence, and preferably at most 20%, yet more preferably at most 10% and most preferably at most 5% (by dry weight) of the sample is contaminating antibodies. A "purified antibody composition" means that at least 99% of the antibodies in the composition are directed against the desired Strep-Tag II sequence SEQ ID NO:9.

The antibody of the invention is able to bind a peptide having a sequence SEQ ID NO:9 which is isolated or which is part of a fusion polypeptide. In a preferred embodiment, the antibody of the invention binds the peptide sequence SEQ ID NO: 9 which is included in a fusion polypeptide. In this case, the said peptide sequence can be located at NH2-terminus, COOH-terminus or inside the said fusion polypeptide. In a more preferred embodiment, the antibody of the invention binds a peptide sequence SEQ ID NO: 9 which is located at the COOH terminus of said fusion polypeptide.

In the context of the present invention, an antibody is said to "recognize" or "bind" a peptide having a define sequence if said antibody has an affinity constant $K_a$ (which is the inverted dissociation constant, i.e. $1/K_d$) higher than $10^6$ $M^{-1}$, preferably higher than $10^7$ $M^{-1}$, more preferably higher than $10^9$ $M^{-1}$ for said peptide. Also, in the context of the present invention, an antibody is said to "specifically bind" or to "specifically recognize" a peptide if said antibody has an affinity constant $K_a$ higher than $10^6$ $M^{-1}$, preferably higher than $10^7$ $M^{-1}$, more preferably higher than $10^9$ $M^{-1}$ for said peptide and has an affinity constant $K_a$ lower than $10^4$ $M^{-1}$ for all the other peptide.

The affinity constant which is used to characterize the binding of antibodies (Ab) to a peptide or an antigen (Ag) is the inverted dissociation constant defined as follows:

$$Ab + Ag \rightleftharpoons AbAg$$

$$K_a = \frac{[AbAg]}{[Ab]\,[Ag]} = \frac{1}{K_d}$$

This affinity can be measured for example by ELISA, Biacore, equilibrium dialysis or by fluorescence quenching, technologies being routinely used in the art.

In a preferred embodiment, the antibody of the invention binds the peptide having the sequence SEQ ID NO:9 with a $K_D$ of less than $10^{-9}$ M, preferably from less than $10^{-10}$ M. In another preferred embodiment, the antibody of the invention binds the peptide having the sequence SEQ ID NO:10 (SAWSHPQFEK) with a $K_D$ of less than $10^{-9}$ M, preferably from less than $10^{-10}$ M. In another preferred embodiment, the antibody of the invention binds the peptide having the sequence SEQ ID NO:11 (AGWSHPQFEKGGGSGGGSGGGSWSHPQFEK) with a $K_D$ of less than $10^{-9}$ M, preferably from less than $10^{-10}$ M.

In a preferred embodiment of the invention, the antibody of the invention is tagged with a detectable marker, preferably a fluorescent or a luminescent marker. Examples of detectable markers / labels include various enzymes, prosthetic groups, fluorescent materials, luminescent materials, bioluminescent materials, and radioactive materials. Examples of suitable enzymes include horseradish peroxidase, alkaline phosphatase, beta-galactosidase, or acetylcholinesterase. Examples of suitable prosthetic group complexes include streptavidin/biotin and avidin/biotin. Examples of suitable fluorescent materials include umbelliferone, fluorescein, fluorescein isothiocyanate, rhodamine, dichlorot[pi] azinylamine fluorescein, dansyl chloride or phycoerythrin. Example of a luminescent material includes luminol. Examples of bioluminescent materials include luciferase, luciferin, and aequorin. And examples of suitable radioactive material include $^{125}$I, $^{131}$I, $^{35}$S or $^{3}$H.

## Hybridoma of the invention

In a preferred embodiment, the antibody of the invention is produced by the hybridoma deposited on November 27, 2012 at the COLLECTION NATIONALE DE CULTURES DE MICROORGANISMES (CNCM) under the number CNCM I-4703.

The hybridoma CNCM I-4703 has been obtained by immunizing a mouse with the recombinant ectodomain of glycoprotein E2 of GB virus (GBV-B), containing a double Strep-Tag of SEQ ID NO:11 (two Strep-Tags separated by a linker peptide).

In another aspect, the invention targets the hybridoma which has been deposited on November 27, 2012 at the COLLECTION NATIONALE DE CULTURES DE MICROORGANISMES (CNCM) of INSTITUT PASTEUR under the number CNCM I-4703.

## Use of the antibody of the invention

The antibody of the invention can be used for example in technologies necessitating high affinity Strep-Tag recognition, for example Western Blotting or Immunohistochemistry methods, or in order to immobilize Strep-tagged fusion proteins on Biacore sensor chips for performing Surface Plasmon Resonance (SPR)-mediated protein interaction studies. It is also possible to use said antibody so as to purify tagged-recombinant proteins, for example by means of an affinity column or in immunoprecipitation experiments.

In particular, the antibody of the invention can be used for purification, detection, immobilization or separation of a recombinant gene product. It can be used for rapid isolation of this gene product, (preferentially under standardized high-throughput conditions), and for the characterization of its biochemical activity and identification of interaction with partners. For all these applications, the antibody of the invention can be used to stably immobilize said gene product on a solid support.

Therefore, in a preferred embodiment, the antibody of the invention is itself immobilized on a solid support. Said solid support can be any support useful in biochemistry or immunochemistry. It is for example a magnetic bead, a microtiter plate, a dipstick support, an affinity column, or a Biocore sensor chip.

In a particular aspect, the present invention targets a solid support (e.g., the above-mentioned ones) coated with the antibody of the invention. Coating methods are well-known in the art Lafaye et al, Res Immunol, 1995.

Protocols for performing purification, detection, immobilization or separation of a recombinant gene product are well-known in the art. One can cite - without being exhaustive: western blots, immunostaining, immunoprecipitations and /or biacore sensor chip. These technologies are described for example in Schmidt TGM and Skerra A., Nature Protocols 2007, and in Sambrook, Fritsch and Maniatis -"Molecular Cloning - A Laboratory Manual" Second Edition Cold Spring Harbor Laboratory, 1989). They do not need to be described in details herein.

In a particular aspect, the present invention is drawn to a method for immobilizing a recombinant polypeptide containing the peptide of sequence SEQ ID NO: 9, said peptide sequence being preferably located at the NH2 terminus or at the COOH terminus of said recombinant polypeptide, on a solid support, said method comprising the step of incubating i) the antibody of the invention with a solid support, in appropriate conditions so that the antibody becomes bound to the solid support, and ii) said recombinant polypeptide with the antibody of the invention bound to the solid support. These detection assays are well-described in Harlow E., Lane D.: Antibodies, a laboratory Manual, Cold Spring Harbor Laboratory, 1988.

This method can be used for example for screening for a ligand of said recombinant polypeptide or for detecting the presence of a recombinant polypeptide in a cell lysate.

This screening method comprises preferably the step of incubating said recombinant polypeptide with a solid support coated with the antibody of the invention, and with a putative ligand.

This method is reduced to practice for example by means of:

- an immunoprecipitation assay (the antibody of the invention being immobilized on a solid bead, thereafter incubated with a cell lysate or a solution containing said recombinant polypeptide, the fixed recombinant polypeptides being finally separated from the bead in appropriate conditions well-known in the art),

- a biacore sensor assay (the antibody of the invention is used to immobilize said recombinant polypeptide on a chip, and association between said recombinant polypeptide and a potential interaction partner is assessed by Surface Plasmon Resonance).

Surface plasmon resonance (SPR) is the collective oscillation of valence electrons in a solid stimulated by incident light. The resonance condition is established when the frequency of light photons matches the natural frequency of surface electrons oscillating against the restoring force of positive nuclei. SPR is the basis of many standard tools for measuring real-time interaction analysis. It is the fundamental principle behind many color-based biosensor applications and different lab-on-a-chip sensors. This assay is well described in Schmidt TGM and Skerra A., Nature Protocols 2007.

Said screening method enables to detect any ligand of said recombinant polypeptide. For example, said ligand can be an antibody recognizing an epitope of said recombinant polypeptide, or, when said polypeptide is an enzyme, a substrate that binds the active site thereof, or, when said polypeptide is a receptor, an agonist or an antagonist thereof, etc.

In another particular aspect, the present invention is drawn to a method for detecting in a sample a recombinant polypeptide containing the peptide of sequence SEQ ID NO: 9, or cells expressing said recombinant polypeptide, said peptide sequence being preferably located at the NH2 terminus or at the COOH terminus of said recombinant polypeptide, comprising the step of incubating said sample with the antibody of the invention, as defined above.

Preferably, said sample is a lysate of cells expressing said recombinant polypeptide, or any solution (cell supernatant, biologic fluid, etc.) containing said recombinant polypeptide. Said lysate is preferably obtained by breaking the cells by using mild periplasmic extractions such as French press or ultrasounds.

This method is for example:

- a western blot assay (the recombinant polypeptide present in a cell lysate or in a solution being immobilized on a membrane, the said membrane being thereafter incubated with the antibody of the invention, preferably labeled, in appropriate conditions well-known in the art),

- an ELISA assay (the recombinant polypeptide being immobilized on a microtiter plate, the said plate being thereafter incubated with the antibody of the invention, preferably labeled, in appropriate conditions well-known in the art),

- an immunohistochemistry assay (the recombinant antibody, preferably labeled, being used to stain a sample containing fixed cells or tissues expressing the recombinant polypeptide),

- a flow cytometry assay (the recombinant antibody, preferably labeled, being used to stain a sample containing fixed or living cells expressing the recombinant polypeptide, in appropriate conditions well-known in the art),

This detecting method comprises preferably the step of incubating said sample with a solid support coated with the antibody of the invention.

In this case, this method is for example:

- an ELISA assay (the antibody of the invention being immobilized on a microtiter plate, the said plate being thereafter incubated with the cell lysate or the solution containing the recombinant polypeptide in appropriate conditions well-known in the art),

- an immunoprecipitation assay (the antibody of the invention being immobilized on a solid bead, thereafter incubated with a cell lysate or a solution containing said recombinant polypeptide, the fixed polypeptides being finally detected by western-blot, mass spectrometry or with any appropriate method well-known in the art).

These detection assays are well-described in Sambrook, Fritsch and Maniatis -"Molecular Cloning-A Laboratory Manual" Second Edition Cold Spring Harbor Laboratory, 1989.

Any other detecting methods requiring the use of an antibody are herein encompassed, although not mentioned.

Both the presence and the amount of said recombinant polypeptide in said sample can be determined thanks to these methods.

In some of these methods, it is necessary to label the antibody of the invention with a detectable marker, preferably a fluorescent or a luminescent marker, as disclosed above.

In another aspect, the present invention is drawn to a method for purifying a recombinant polypeptide containing the peptide of sequence SEQ ID NO: 9, said peptide sequence being preferably located at the NH2 terminus or at the COOH terminus of said recombinant polypeptide, comprising the step of incubating said recombinant polypeptide with the antibody of the invention, as defined above.

This method is for example:

- a flow cytometry assay (the recombinant antibody being labeled and used to stain a sample containing living cells expressing the recombinant polypeptide, preferably at their surface, in appropriate conditions well-known in the art),

- an immunoprecipitation assay (the antibody of the invention being immobilized on a solid bead, thereafter incubated with a cell lysate or a solution containing said recombinant polypeptide, the fixed recombinant polypeptides being finally separated from the bead in appropriate conditions well-known in the art),

- a purification method using an affinity column (the antibody of the invention being immobilized on an affinity column, thereafter incubated with a cell lysate or a solution containing said recombinant polypeptide, the fixed recombinant polypeptides being finally eluted from the column in appropriate conditions well-known in the art).

These assays are well-described in Sambrook, Fritsch and Maniatis -"Molecular Cloning - A Laboratory Manual" Second Edition Cold Spring Harbor Laboratory, 1989.

In another aspect, the present invention is drawn to a method for purifying and /or sorting cells expressing, either at their surface or intracellularly, a recombinant polypeptide containing the peptide of sequence SEQ ID NO: 9, said peptide sequence being preferably located at the NH2 terminus or at the COOH terminus of said recombinant polypeptide, comprising the step of incubating said cells with the antibody of the invention, as defined above.

The cells expressing said recombinant polypeptide can be either alive or inactivated.

If required, the cells are inactivated preferably by means of a chemical and / or a heating treatment. These treatments are well known in the art. For example, an inactivating chemical agent is preferably a fixative agent, for example chosen in the group consisting of: aldehyde (such as formaldehyde, glutaraldehyde, and paraformaldehyde), alcohols (such as methanol, ethanol), acetone, oxidizing agents (such as osmium tetroxide, potassium dichromate, chromic acid, and potassium permanganate) and the like.

For an extracellular staining (i.e., if the recombinant polypeptide is expressed at the surface of the cells), the antibody of the invention can be incubated with the living or inactivated cells in appropriate conditions, preferably on ice.

For an intracellular staining, the cells are preferably previously inactivated and then permeabilized, before the antibody of the invention is added.

Such a method is for example a flow cytometry assay (the recombinant antibody, preferably labeled, being put in contact with a sample containing living or inactivated cells expressing the recombinant polypeptide, preferably at their surface, in appropriate conditions well-known in the art). This technic allows using heterogeneous mixtures of biological cells (e.g., whole blood samples) and not isolating and/or purifying the cells before performing the staining. Also, it can be easily performed on living cells (so that the natural conformation of the strep-tag peptide is not affected). Flow cytometry is a broadly used scientific technic for purifying and sorting of individual cells.

These assays are well-described in Sambrook, Fritsch and Maniatis -"Molecular Cloning - A Laboratory Manual" Second Edition Cold Spring Harbor Laboratory, 1989.

In a preferred embodiment of the invention, the antibody of the invention is labeled with a detectable marker, preferably a fluorescent or a luminescent marker, as disclosed above.

**Kit of the invention**

In a final aspect, the present invention is drawn to a kit comprising:

- the antibody of the invention or
- the solid support of the invention, which is coated with the antibody of the invention.

This kit can be used in any of the above-mentioned method.

This kit may also contain any reagent adapted for chemical or immunological reaction with the antibody of the invention, so as to detect its presence in the tested sample. It may for example contain a revealing agent that will react with the label carried by the antibody of the invention, or secondary antibodies, which are preferably labelled.

It may also contain instructions for using said kit in an appropriate manner.

This kit can be used in particular in an immunoprecipitation assay, in a biacore sensor assay, in a western blot assay, in an ELISA assay, in an immunohistochemistry assay, in a flow cytometry assay as well as in a purification method using an affinity column.

## EXAMPLES

### Example I: Generation of the antibody of the invention

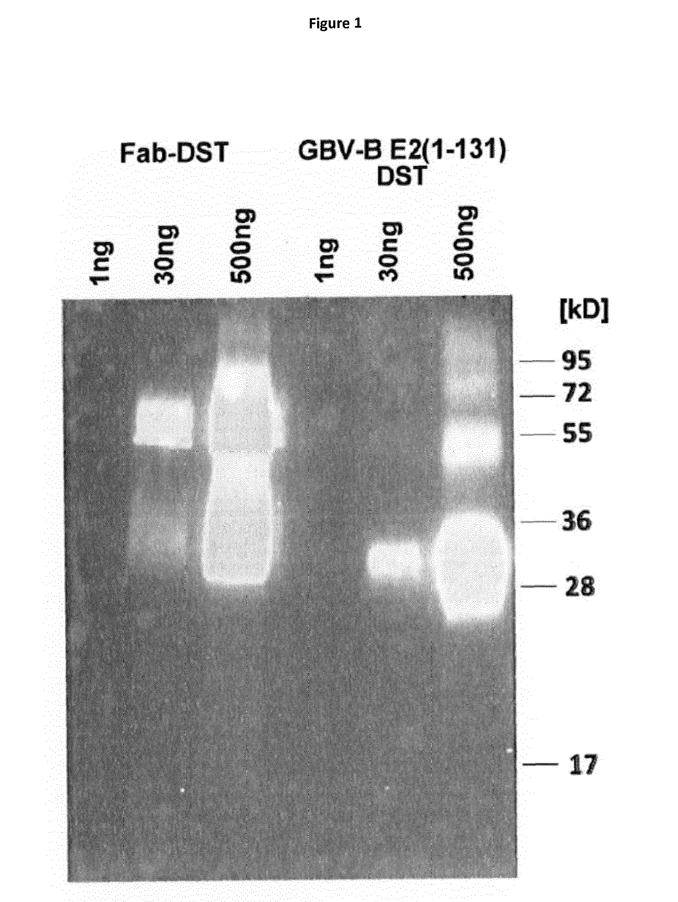

Mouse immunization experiments have been conducted with the recombinant ectodomain of glycoprotein E2 of GB virus (GBV-B), a tamarin virus that is closely related to the human hepatitis C virus. For purification purposes, this protein contained double Strep-Tag - i.e., two Strep-Tags separated by the linker peptide GGGSGGGSGGGS. Several antibodies were obtained, recognizing specifically the tagged GBV-B protein. The antibody C23.21 was obtained along with a number of other antibodies. The specificity of C23.21 became evident once it was realized that it reacted with unrelated recombinant proteins that had in common only the presence of the Strep-Tag, as shown in a Western Blot analysis (figure 1).

For obtaining figure 1, Western Blotting using 1$\mu$g/mL C23.21 monoclonal antibody purified by Protein G affinity purification, 40ng/mL anti-mouse secondary antibody conjugated to horseradish peroxidase (HRP) and ECL chemiluminescent detection system (10 seconds exposure) was performed. Lanes 1-3 contains control Fab fused to the double Strep-Tag of sequence NH2 - AGWSHPQFEKGGGSGGGSGGGSWSHPQFEK - COOH (SEQ ID NO:11), and lanes 4-6 contains GBV-B E2 fused to the same double Strep-Tag.

As shown on figure 1, both proteins are recognized by C23.21 with similar efficiency.

It can be concluded therefore that C23.21 recognizes efficiently the double Strep-Tag which does not contain the SA amino acids upstream the Strep-Tag II sequence (but apparently a "AG" sequence). The binding of C23.21 to its target sequence seems thus to be independent of the presence of the two amino acids "SA" in the linker, in contrary to the commercial antibodies available so far.

### Example II: Characterization of the antibody of the invention

#### *Hybridoma production*

Balb/C mice were immunized subcutaneously (s.c.) with 10 $\mu$g of the recombinant ectodomain of glycoprotein E2 of GB virus (GBV-B, SEQ ID NO:12) with 100 $\mu$l of complete Freund's adjuvant. Five s.c. booster injections, in incomplete Freund's adjuvant, were given 1, 3, 5 and 7 weeks later. Fifteen days later, mice received the last intra-peritoneal booster of antigen (10 $\mu$g). Splenocytes were collected three days later and immortalized by fusion with mouse myeloma cells (x63Ag8-653). Cells grown on 24-well plates were screened by ELISA. Hybridomas from positive wells were cloned by limiting dilution and plated on microtiter plates. Positive clones identified by ELISA were expanded and used to inject mice for ascites production.

#### *Results*

The antibody C23.21 belongs to the murine IgG1 subtype with a Kappa light chain.

It recognizes its antigen SEQ ID NO:9 (WSHPQFEK) via the specific sequence present in the Strep Tag with a dissociation constant of $1.4 \times 10^{-10}$M (as determined with an ELISA assay, cf Friguet et al, J. Immunol. Methods 1985).

It recognizes another fusion protein namely VHH-E9 containing the strep-tag sequence of SEQ ID NO: 13 at the C terminus with a dissociation constant of $9.1 \times 10^{-10}$M.

The antibody C23.21 has a light chain sequence comprising the three following Complementary Determining Regions (CDRs):

i) CDR1 (LC-CDR1): ESVDSYGKSF (SEQ ID NO:1);

ii) CDR2 (LC-CDR2): RAS (SEQ ID NO:2);

iii) CDR3 (LC-CDR3): QQNNEDPWT (SEQ ID NO: 3);

The antibody C23.21 has a heavy chain sequence comprising the three following Complementary Determining Regions (CDRs):

i) CDR1 (HC-CDR1): GYTFTNYY (SEQ ID NO: 4);

ii) CDR2 (HC-CDR2): LNPNNGDT (SEQ ID NO: 5);

iii) CDR3 (HC-CDR3): ARTGRYEENAMDY (SEQ ID NO: 6).

**Example III: Use of the antibody of the invention in an ELISA and a Western Blot**

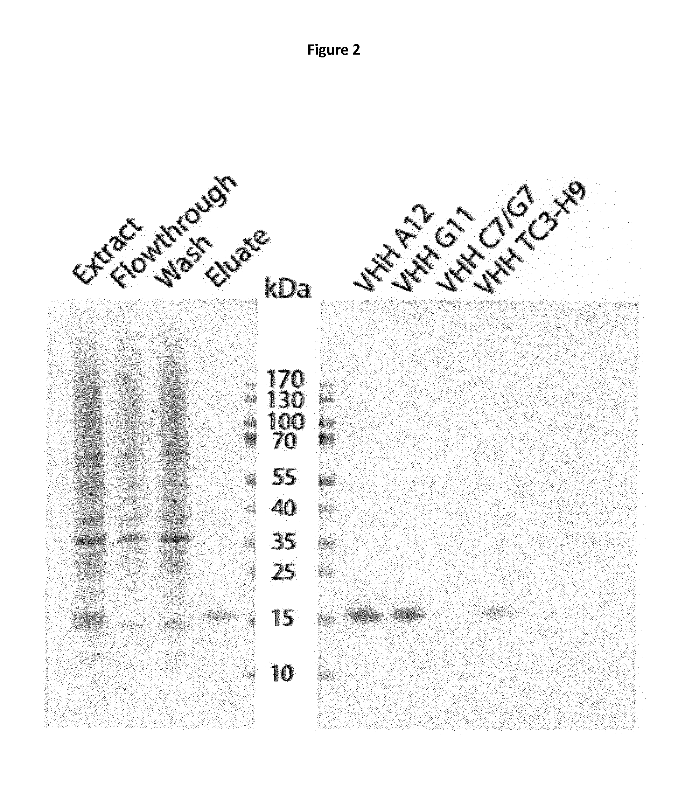

Various camelid antibody fragments (VHH) have been cloned in frame with the Strep-Tag II of SEQ ID NO: 13 (LESAWSHPQFEK) in a periplasm of *E.coli* and purified using a streptactin affinity column (see figure 2).

*Material and methods*

The coding sequences of the selected VHHs were cloned in a modified pASK-IBA2 vector (Genosys IBA) containing the NcoI and NotI restriction sites. pASK-IBA2 allows the periplasmic expression of a strep-tag II at the C-terminal end in frame with VHH and it was mutagenenesised at the N-terminal of strep-tag II for introducing NotI restriction site. Transformed *E. coli* XL2-Blue ultracompetent cells expressed VHHs in the periplasm after induction by anhydrotet-racycline ($200 \mu$g/l) for 16 h at 20°C. Cells were resuspended in 100mM Tris-Cl pH 8, 150mM NaCl, 1 mM EDTA, then lysed by polymyxin B sulfate (1mg/ml Fulka) 1 h at 4°C, the periplasmic extract was then obtained after centrifugation. Purified VHHs were obtained by Strep-Tactin Sepharose column (IBA) according to the manufactuer's instructions.

*Results*

**Figure 2** discloses a SDS-PAGE analysis of crude extract, flow through, wash and elution fractions of the purification of VHH E9 (left) and purified VHH A12, G11, C7/G7 (right).

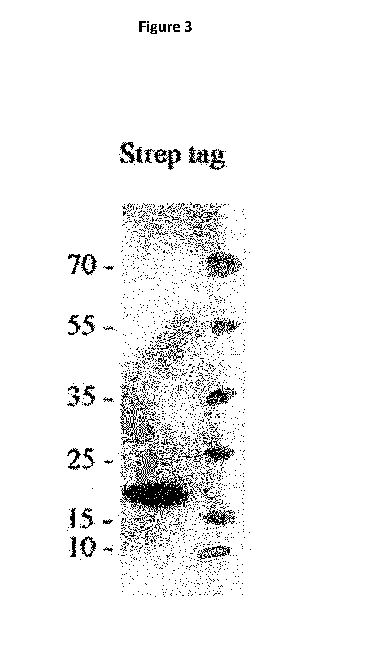

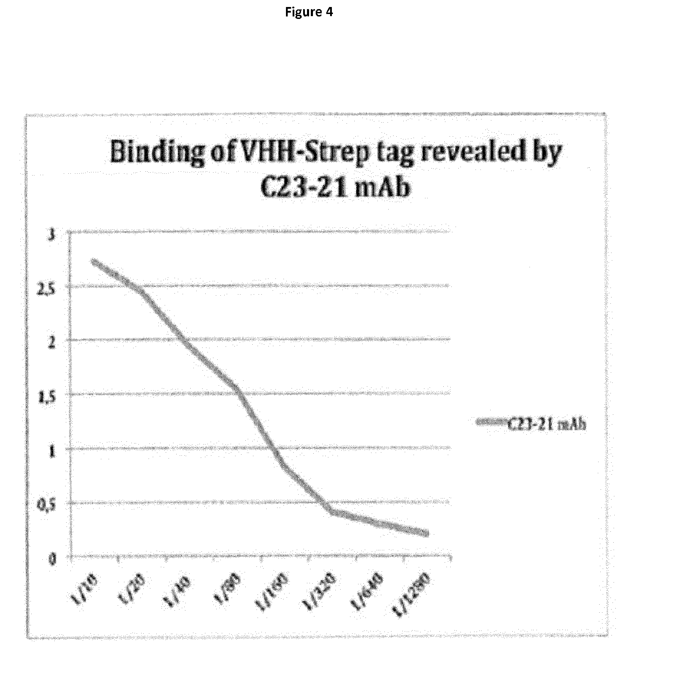

C23.21 was used to detect the purified VHH-tagged in ELISA and Western Blot analysis respectively (figures 3 and 4 respectively).

• **ELISA**

A modified version of a standard ELISA was used to test for the presence of antibodies in culture supernatants. Microtiter plates (Nunc, Denmark) were coated by incubation overnight at 4°C with 1 $\mu$g/ml of GFAP antigen diluted in PBS. Plates were washed four times with buffer A (0.1% Tween 20 in PBS), and anti-GFAP VHH E9 was diluted in buffer B (0.5% gelatin in buffer A). The plates were incubated for 2 hours at 37°C, washed again and C23-21 mAb (1$\mu$g/ml) was added for 1 hour at 37°C. Then after washing with buffer A, horseradish peroxydase-labeled rabbit anti-mouse antibodies (Bio-Rad Pasteur, France) were added. Then, the plates were washed with buffer A, and freshly prepared 0.2 % orthophenylenediamine (Dakopatts A/S, Glostrup, Denmark), 0.03% $H_2O_2$ in 0.1 M citrate buffer, pH 5.2, were added to each well. The peroxidase reaction was stopped by adding 3 M HCl, and the optical density measured at 490 nm.

• **Western Blot**

Strep-tagged VHH was suspended in PBS and heat inactivated at 56°C during 30 minutes. To an aliquote (5$\mu$l) of Strep-tagged VHH an equal volume of gel loading buffer was added and then treated at 100°C for 5min. Following separation by SDS 12% polyacrylamide gel electrophoresis (PAGE), semi-dry transfer onto Hybond-C (Amersham) and western blotting were carried out. Immunoblotting of membrane was accomplished with anti-strep tag C23-21 mAb (1 μg/ml) and was respectively revealed by horseradish peroxydase-labeled rabbit anti-mouse antibodies (Bio-Rad Pasteur, France).

**Figure 3** discloses a Western Blot result of a purified Strep-Tagged VHH revealed with the monoclonal antibody of the invention.

**Figure 4** discloses the binding of Strep-tagged VHH E9 to its antigen GFAP in ELISA. The monoclonal antibody of the invention C23.21 was used as a secondary antibody to detect binding.

These results demonstrate that C23.21 recognizes both the single and the double Strep-Tag and is entirely independent of the upstream linker sequence (AG vs. SA in the double Strep-Tag and the single Strep-Tag respectively).

**Bibliographic references**

Arnau J, Lauritzen C, Petersen GE, Pedersen J. Current strategies for the use of affinity tags and tag removal for the purification of recombinant proteins. Protein Expr Purif. 2006 Jul;48(1):1-13.

Bird et al. (1988) Science 242 423-426

CHOTHIA et al., J. Mol. Biol., vol.196, p: 901-17, 1987;

CHOTHIA et al., Nature, vol.342, p: 878-83, 1989

Cole et al., Methods Enzymol, 121; 140-67, 1986

Friguet et al, J. Immunol. Methods 1985, Mar 18;77(2):305-19.

Huse et al., Science 246; 1275, 1989

Junttila, M.R., Saarinen, S., Schmidt, T., Kast, J. & Westermarck, J. Single-step Strep-tags purification for the isolation and identification of protein complexes from mammalian cells. Proteomics 5, 1199-1203 (2005)

Kohler and Milstein. Nature 256; 495- 497 (1975)

Kozbor et al, Immunol Today 4 72 (1983)

Lafaye et al, Res Immunol, 1995 Jul-Aug;146(6):373-82

Perruchini et al, Acta Neuropathologica 2009 Nov;118(5):685-95.

Schmidt, T.G., Koepke, J., Frank, R. & Skerra, A. Molecular interaction between the Strep-tag affinity peptide and its cognate target, streptavidin. J. Mol. Biol. 255, 753-766 (1996).

Schmidt TG, Skerra A. The Strep-tag system for one-step purification and high-affinity detection or capturing of proteins. Nat Protoc. 2007;2(6):1528-35.

Skerra A, Schmidt TG. Applications of a peptide ligand for streptavidin: the Strep-tag. Biomol. Eng. 1999 Dec 31;16(1-4):79-86.

Skerra, A. & Schmidt, T.G. Use of the Strep-tag and streptavidin for detection and purification of recombinant proteins. Methods Enzymol. 326, 271-304 (2000).

Terpe K. Overview of tag protein fusions: from molecular and biochemical fundamentals to commercial systems. Appl Microbiol Biotechnol. 2003 Jan;60(5):523-33.

**Claims**

1.  An antibody or a fragment thereof, which binds a peptide having the sequence represented by SEQ ID NO: 9 (Strep-Tag II), said antibody comprising six Complementary Determining Regions (CDRs) consisting of SEQ ID NO:1-6.

2. The antibody of claim 1, comprising:

   a) a light chain comprising three CDRs having the following amino acid sequences:

   i) the light chain CDR1 (LC-CDR1): ESVDSYGKSF (SEQ ID NO:1);
   ii) the light chain CDR2 (LC-CDR2): RAS (SEQ ID NO:2);
   iii) the light chain CDR3 (LC-CDR3): QQNNEDPWT (SEQ ID NO: 3);
   and

   b) a heavy chain comprising three CDRs having the following amino acid sequences:

   i) the heavy chain CDR1 (HC-CDR1): GYTFTNYY (SEQ ID NO: 4);
   ii) the heavy chain CDR2 (HC-CDR2): LNPNNGDT (SEQ ID NO: 5);
   iii) the heavy chain CDR3 (HC-CDR3): ARTGRYEENAMDY (SEQ ID NO: 6).

3. The antibody of claim 1 or 2, comprising a light chain variable region ($V_L$) having the amino acid sequence SEQ ID NO: 7.

4. The antibody of any one of claim 1 to 3, comprising a heavy chain variable region ($V_H$) having the amino acid sequence SEQ ID NO: 8.

5. The antibody of any one of claim 1 to 4, comprising a $V_L$ having the amino acid sequence SEQ ID NO: 7 and a $V_H$ having the amino acid sequence SEQ ID NO: 8.

6. The antibody of any one of claim 1 to 5, wherein said peptide sequence SEQ ID NO: 9 is part of a fusion polypeptide.

7. The antibody of claim 6, wherein said peptide sequence SEQ ID NO: 9 is located at the NH2 terminus or at the COOH terminus of said fusion polypeptide.

8. The antibody of any one of claim 1 to 7, wherein said antibody is a monoclonal antibody.

9. The antibody of any one of claim 1 to 8, said antibody being produced by the hybridoma deposited on November 27, 2012 at the COLLECTION NATIONALE DE CULTURES DE MICROORGANISMES (CNCM) under the number CNCM I-4703.

10. The antibody of any one of claim 1 to 9, wherein said antibody binds the peptide of SEQ ID NO:9 with a $K_D$ of less than $10^{-9}$ M, preferably from less than $10^{-10}$ M.

11. The antibody of any one of claim 1 to 10, which is immobilized on a solid support.

12. The antibody of claim 11, wherein said solid support is a magnetic bead, a microtiter plate, an affinity column, or a Biacore sensor chip.

13. A method for detecting in a sample a recombinant polypeptide containing the peptide of sequence SEQ ID NO: 9, or cells expressing said recombinant polypeptide, comprising the step of incubating said sample with the antibody defined in any one of claim 1 to 12.

14. A method for screening for a ligand of a recombinant polypeptide containing the peptide of sequence SEQ ID NO: 9, comprising the step of incubating said recombinant polypeptide with the antibody defined in any one of claim 1 to 12, and a putative ligand.

15. The hybridoma which has been deposited on November 27, 2012 at the COLLECTION NATIONALE DE CULTURES DE MICROORGANISMES (CNCM) of INSTITUT PASTEUR under the number CNCM I-4703.

Figure 1

**Figure 2**

Figure 3

Strep tag

Figure 4

Binding of VHH-Strep tag revealed by C23-21 mAb

Europäisches
Patentamt

European
Patent Office

Office européen
des brevets

## EUROPEAN SEARCH REPORT

**Application Number**

EP 13 30 6527

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | MELISSA R. JUNTTILA ET AL: "Single-stepStrep-tag&#xFFFD; purification for the isolation and identification of protein complexes from mammalian cells", PROTEOMICS, vol. 5, no. 5, 1 April 2005 (2005-04-01), pages 1199-1203, XP055106407, ISSN: 1615-9853, DOI: 10.1002/pmic.200400991 * figure 1 * * page 1201, left-hand column, paragraph 1 * | 1-15 | INV. C07K16/12 G01N33/53 |
| Y | SCHMIDT THOMAS G M ET AL: "The Strep-tag system for one-step purification and high-affinity detection or capturing of proteins", NATURE PROTOCOLS, NATURE PUBLISHING GROUP, GB, vol. 2, no. 6, 1 January 2007 (2007-01-01), pages 1528-1535, XP001536704, ISSN: 1750-2799, DOI: 10.1038/NPROT.2007.209 * abstract * * figure 2 * * Box 2; page 1521 * | 1-15 | |

-/--

TECHNICAL FIELDS
SEARCHED (IPC)

C07K
G01N

The present search report has been drawn up for all claims

| Place of search | Date of completion of the search | Examiner |
|---|---|---|
| Munich | 11 March 2014 | Irion, Andrea |

CATEGORY OF CITED DOCUMENTS

X : particularly relevant if taken alone
Y : particularly relevant if combined with another
　document of the same category
A : technological background
O : non-written disclosure
P : intermediate document

T : theory or principle underlying the invention
E : earlier patent document, but published on, or
　after the filing date
D : document cited in the application
L : document cited for other reasons

........................................................................

& : member of the same patent family, corresponding
　document

EPO FORM 1503 03.82 (P04C01)

Europäisches
Patentamt

European
Patent Office

Office européen
des brevets

# EUROPEAN SEARCH REPORT

Application Number

EP 13 30 6527

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | Christian Johannes Gloeckner ET AL: "Strep/FLAG Tandem Affinity Purification (SF-TAP) to Study Protein Interactions" In: "Current Protocols in Protein Science", 1 May 2001 (2001-05-01), John Wiley & Sons, Inc., Hoboken, NJ, USA, XP055106608, ISBN: 978-0-47-114086-3 DOI: 10.1002/0471140864.ps1920s57, * page 19.20.17, right-hand column, paragraph 3 * | 1-15 | |
| Y | THOMAS G.M. SCHMIDT ET AL: "Development of the Twin-Strep-tag and its application for purification of recombinant proteins from cell culture supernatants", PROTEIN EXPRESSION AND PURIFICATION, vol. 92, no. 1, 1 November 2013 (2013-11-01), pages 54-61, XP055106609, ISSN: 1046-5928, DOI: 10.1016/j.pep.2013.08.021 * abstract * * page 55, right-hand column, paragraph 7 * * page 56, left-hand column, paragraph 3-4 * | 1-15 | TECHNICAL FIELDS SEARCHED (IPC) |
| Y | WO 2010/078966 A1 (APOGENIX GMBH [DE]; HILL OLIVER [DE]; BRANSCHAEDEL MARCUS [DE]; GIEFFE) 15 July 2010 (2010-07-15) * page 41, lines 19-22 * * page 44, line 27 - page 45, line 8 * | 1-15 | |

-/--

The present search report has been drawn up for all claims

| Place of search | Date of completion of the search | Examiner |
|---|---|---|
| Munich | 11 March 2014 | Irion, Andrea |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

## EUROPEAN SEARCH REPORT

Application Number

EP 13 30 6527

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WO 2013/160453 A2 (IBA GMBH [DE]) 31 October 2013 (2013-10-31) * paragraph [0020] * * paragraph [0041] * * figures 4,5 * | 1-15 | |
| Y | MINORU FUNAKOSHI ET AL: "Small epitope-linker modules for PCR-based C-terminal tagging in Saccharomyces cerevisiae", YEAST, vol. 26, no. 3, 1 March 2009 (2009-03-01), pages 185-192, XP055106880, ISSN: 0749-503X, DOI: 10.1002/yea.1658 * abstract * * figure 1 * * table 2 * * page 188, left-hand column, paragraph 1 * | 1-15 | |

TECHNICAL FIELDS
SEARCHED    (IPC)

The present search report has been drawn up for all claims

| Place of search | Date of completion of the search | Examiner |
|---|---|---|
| Munich | 11 March 2014 | Irion, Andrea |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 13 30 6527

This annex lists the patent family members relating to the patent documents cited in the above-mentioned European search report.
The members are as contained in the European Patent Office EDP file on
The European Patent Office is in no way liable for these particulars which are merely given for the purpose of information.

11-03-2014

| Patent document cited in search report | Publication date | Patent family member(s) | Publication date |
|---|---|---|---|
| WO 2010078966 A1 | 15-07-2010 | EP 2382236 A1<br>JP 2012514616 A<br>US 2012041181 A1<br>WO 2010078966 A1 | 02-11-2011<br>28-06-2012<br>16-02-2012<br>15-07-2010 |
| WO 2013160453 A2 | 31-10-2013 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 4816567 A, Cabilly
- US 4816397 A, Boss
- US 4946778 A, Ladner
- US 4676980 A
- US 6121424 A
- US 5223409 A
- WO 9218619 A
- WO 9117271 A

### Non-patent literature cited in the description

- **TERPE K.** *Appl. Microbiol. Biotechnol.,* 2003
- **ARNAU J. et al.** *Protein Expr. Purif.,* 2006
- **SKERRA A ; SCHMIDT TG.** *Biomol. Eng.,* 1999
- **SKERRA A ; SCHMIDT TG.** *Methods Enzymol.,* 2000
- **SCHMIDT T. ; SKERRA A.** *Nature protocols,* 2007
- **JUNTTILA M.R. et al.** *Proteomics,* 2005
- **SCHMIDT TG et al.** *J.Mol. Biol.,* 1996
- **SCHMIDT T. ; SKERRA A.** IBA antibodies, the Box 2. *Nature protocols,* 2007
- **PERRUCHINI et al.** *Acta Neuropathologica,* 2009
- **CHOTHIA et al.** *J. Mol. Biol.,* 1987
- **CHOTHIA et al.** *Nature,* 1989
- **KOHLER ; MILSTEIN.** *Nature,* 1975, vol. 256, 495-497
- **KOZBOR et al.** *Immunol Today,* 1983, vol. 4, 72
- **COLE et al.** *Methods Enzymol,* 1986, vol. 121, 140-67
- **HUSE et al.** *Science,* 1989, vol. 246, 1275
- **BIRD et al.** *Science,* 1988, vol. 242, 423-426
- **LAFAYE et al.** *Res Immunol,* 1995
- **SCHMIDT TGM ; SKERRA A.** *Nature Protocols,* 2007
- **SAMBROOK ; FRITSCH ; MANIATIS.** Molecular Cloning - A Laboratory Manual. Cold Spring Harbor Laboratory, 1989
- **HARLOW E. ; LANE D.** Antibodies, a laboratory Manual. Cold Spring Harbor Laboratory, 1988
- **SAMBROOK ; FRITSCH ; MANIATIS.** Molecular Cloning-A Laboratory Manual. Cold Spring Harbor Laboratory, 1989
- **FRIGUET et al.** *J. Immunol. Methods,* 1985
- **ARNAU J ; LAURITZEN C ; PETERSEN GE ; PEDERSEN J.** Current strategies for the use of affinity tags and tag removal for the purification of recombinant proteins. *Protein Expr Purif.,* 2006, vol. 48 (1), 1-13
- **BIRD et al.** *Science,* 1998, vol. 242, 423-426
- **CHOTHIA et al.** *J. Mol. Biol,* 1987, vol. 196, 901-17
- **CHOTHIA et al.** *Nature,* 1989, vol. 342, 878-83
- **FRIGUET et al.** *J. Immunol. Methods,* 18 March 1985, vol. 77 (2), 305-19
- **JUNTTILA, M.R. ; SAARINEN, S. ; SCHMIDT, T. ; KAST, J. ; WESTERMARCK, J.** Single-step Strep-tags purification for the isolation and identification of protein complexes from mammalian cells. *Proteomics,* 2005, vol. 5, 1199-1203
- **LAFAYE et al.** *Res Immunol,* November 2009, vol. 146 (6), 373-82
- **PERRUCHINI et al.** *Acta Neuropathologica,* vol. 118 (5), 685-95
- **SCHMIDT, T.G. ; KOEPKE, J. ; FRANK, R. ; SKERRA, A.** Molecular interaction between the Strep-tag affinity peptide and its cognate target, streptavidin. *J. Mol. Biol.,* 1996, vol. 255, 753-766
- **SCHMIDT TG ; SKERRA A.** The Strep-tag system for one-step purification and high-affinity detection or capturing of proteins. *Nat Protoc.,* 2007, vol. 2 (6), 1528-35
- **SKERRA A ; SCHMIDT TG.** Applications of a peptide ligand for streptavidin: the Strep-tag. *Biomol. Eng.,* 31 December 1999, vol. 16 (1-4), 79-86
- **SKERRA, A. ; SCHMIDT, T.G.** Use of the Strep-tag and streptavidin for detection and purification of recombinant proteins. *Methods Enzymol.,* 2000, vol. 326, 271-304
- **TERPE K.** Overview of tag protein fusions: from molecular and biochemical fundamentals to commercial systems. *Appl Microbiol Biotechnol.,* January 2003, vol. 60 (5), 523-33